# NEUE EUROPÄISCHE PATENTSCHRIFT

(11) **EP 2 723 659 B2**
(45) Veröffentlichungstag und Bekanntmachung des Hinweises auf die Entscheidung über den Einspruch: **29.05.2019**
(45) Hinweis auf die Patenterteilung: 06.04.2016
(21) Anmeldenummer: 12722306.3
(22) Anmeldetag: 18.05.2012
(51) Int. Cl.: B65G 47/08, B65H 33/18, B65B 35/44, B65B 35/30, B65B 35/24

(54) **VORRICHTUNG UND VERFAHREN ZUM GRUPPIEREN VON GEGENSTÄNDEN**
DEVICE AND METHOD FOR GROUPING ARTICLES
DISPOSITIF ET PROCÉDÉ PERMETTANT DE GROUPER DES OBJETS

(30) Priorität: 27.06.2011 DE 102011105887
(43) Veröffentlichungstag der Anmeldung: 30.04.2014
(73) Patentinhaber: Focke & Co. (GmbH & Co. KG), 27283 Verden (DE)
(72) Erfinder: SCHULTE, Josef, 26871 Aschendorf (DE); PRAHM, Andreas, 26676 Barßel (DE); BRANDHORST, Björn, 48477 Hörstel (DE)
(74) Vertreter: Ellberg, Nils
(86) Internationale Anmeldenummer: PCT/EP2012/002132
(87) Internationale Veröffentlichungsnummer: WO 2013/000527

(56) Entgegenhaltungen:
- EP-A2- 2 165 934
- EP-B1- 1 194 249
- DE-A1-102006 045 087
- GB-A- 1 097 842
- GB-A- 2 454 166
- JP-A- H0 487 929
- JP-A- 2008 073 653
- US-A- 3 563 377

## Beschreibung

Die Erfindung betrifft eine Vorrichtung zum Gruppieren von flachen Gegenständen, insbesondere von Hygieneprodukten wie Windeln, wobei die zu gruppierenden Gegenstände einzeln und nacheinander auf einem Zuführförderer transportierbar und einem Gruppierförderer zuführbar sind, gemäß dem Oberbegriff des Anspruchs 1.

In der Praxis (s. z. B. EP 1 194 249) ist es üblich, dass die zu gruppierenden Gegenstände einzeln und mit Abstand zueinander auf einem Zuführförderer transportiert und von diesem in einen anschließenden (Gruppier-) Förderer mit Fächern für die Gegenstände übergeben werden. Bei dem Gruppierförderer kann es sich beispielsweise um einen Fächerförderer handeln, mit mehreren Fächern für jeweils einen Gegenstand. Aus dem Fächerförderer werden dann im weiteren Verlauf mehrere Gegenstände ausgeschoben unter Bildung einer Gruppe.

Nachteilig an derartigen Lösungen ist der sehr große Platzbedarf für den Fächerförderer. Die großen Abmessungen des Fächerförderers erzwingen in nachteiliger Weise ein entsprechend angepasstes Layout der Verpackungslinie.

Ein weiterer Nachteil besteht darin, dass durch die großen Abmessungen des Fächerförderers die einzelnen Stationen innerhalb der Verpackungslinie bei Störungen nur schwer zugänglich sind. Zudem sind bekannte Fächerförderer nur unter großem Aufwand umzurüsten, sodass diese nur eingeschränkt für Gegenstände unterschiedlicher Größe eingesetzt werden können.

Hiervon ausgehend liegt der Erfindung die Aufgabe zugrunde Vorrichtungen der eingangs genannten Art weiterzuentwickeln, insbesondere im Hinblick auf einen möglichst geringen Platzbedarf.

Zur Lösung dieser Aufgabe weist eine erfindungsgemäße Vorrichtung die Merkmale des Anspruchs 1 auf.

Weitere Einzelheiten der erfindungsgemäßen Vorrichtung sind den Unteransprüchen und der Beschreibung im Übrigen zu entnehmen.

Ein bevorzugtes Ausführungsbeispiel der Erfindung wird nachfolgend anhand der Zeichnung beschrieben. In dieser zeigen:
- Fig. 1: eine Draufsicht auf eine Vorrichtung zum Gruppieren von Gegenständen,
- Fig. 2: ein Vertikalschnitt durch die Vorrichtung entlang Schnittlinie II - II in Fig. 1,
- Fig. 3: ein Vertikalschnitt durch die Vorrichtung entlang Schnittlinie III - III in Fig. 1,
- Fig. 4 und Fig. 5: einen Horizontalschnitt durch die Vorrichtung entlang Schnittlinie IV-IV in Fig. 3 während verschiedener Phasen des Gruppiervorgangs.

Die Erfindung wird nachfolgend anhand einer Vorrichtung zum Gruppieren von Gegenständen 10 beschrieben. Bei den Gegenständen 10 kann es sich um (verpackte) Hygieneprodukte wie Windeln, Binden oder Reinigungstücher handeln.

Im vorliegenden Fall weisen die Gegenstände 10 eine flache Gestalt auf und werden mit Hilfe der Vorrichtung zu einer Produktgruppe 11 zusammengestellt. Innerhalb der Produktgruppe 11 liegen die Gegenstände 10 mit großflächigen Vorder- bzw. Rückseiten aneinander vorzugsweise in Dichtlage an.

Die nachfolgend beschriebene Vorrichtung kann Teil einer Verpackungslinie bzw. Herstellungslinie für die Gegenstände 10 sein. Es werden jedoch nur die Bereiche der Linie beschrieben, die für den Vorgang des Gruppierens von Bedeutung sind.

Zentrales Organ der Vorrichtung ist ein Gruppierförderer 12. Der Gruppierförderer 12 weist aufeinanderfolgende Fächer 13 zur Aufnahme jeweils einer Produktgruppe 11 aus Gegenständen 10 auf.

Im vorliegenden Fall ist der Gruppierförderer 12 als Endlosförderer ausgebildet, mit einem Fördertrum 14, das über Umlenkrollen 15 geführt ist. Eine oder mehrere der Umlenkrollen 15 können mittels eines entsprechenden Antriebs 22 angetrieben werden. Der Antrieb 22 arbeitet vorzugsweise kontinuierlich.

Am Fördertrum 14 sind mit Abstand zueinander Mitnehmer 16 angeordnet, die senkrecht bzw. radial zum Fördertrum 14 angeordnet sind. Durch die Mitnehmer 16 werden die Fächer 13 quer zur Förderrichtung des Gruppierförderers 12 begrenzt, wobei zwei aufeinanderfolgende Fächer 13 durch einen gemeinsamen Mitnehmer 16 voneinander getrennt werden. Die Förderrichtung des Gruppierförderers 12 ist durch Pfeile 17 angedeutet.

Der Abstand zwischen zwei Mitnehmern 16 in Förderrichtung des Gruppierförderers 12 entspricht der entsprechenden Breite der Produktgruppe 11. Der Abstand zwischen den Mitnehmern 16 kann auch so gewählt werden, dass die Produktgruppe 11 geringfügig komprimiert wird.

Die Gruppierung der Gegenstände 10 erfolgt im Bereich eines Obertrums 18 des Gruppierförderers 12. Es ist ein Zuführförderer 19 vorgesehen, mit dem die Gegenstände 10 einzeln und mit Abstand in Transportrichtung zueinander transportiert werden. Die Transportrichtung des Zufuhrförderers 19 ist winklig, insbesondere quer, zum Gruppierförderer 12 ausgerichtet, sodass die Gegenstände 10 in die seitlich offenen Fächer 13 eingeführt werden können.

Im vorliegenden Fall werden die Gegenstände 10 im Bereich des Zuführförderers 19 in der gleichen Relativlage transportiert, in der sie auch in den Fächern 13 des Gruppierförderers 12 angeordnet sind. Die Gegenstände 10 ruhen bzw. stehen mit einer Schmalseite auf dem Obertrum 18 des Gruppierförderers 12. Entsprechend werden die Gegenstände 10 in der gleichen Relativlage und im Wesentlichen höhengleich durch den Zuführförderer 19 zugeführt.

Im Bereich des Zuführförderers 19 werden die Gegenstände 10 beiderseits durch parallel verlaufende Fördertrume von Endlosförderern 20, 21 gehalten. Die Endlosförderer 20, 21 können so konstruiert sein, dass im Bereich des Zuführförderers 19 auch noch eine Änderung der Relativlage der Gegenstände 10, insbesondere eine Drehung, vorgenommen wird, bevor die Gegenstände 10 in der endgültigen Relativlage an den Gruppierförderer 12 übergeben werden. Auch der Zuführförderer 19 wird durch einen Antrieb (nicht gezeigt) angetrieben, vorzugsweise kontinuierlich.

Die Gegenstände 10 werden durch den Zuführförderer 19 einzeln in ein Fach 13 auf dem Obertrum 18 des Gruppierförderers 12 zugeführt. Die Zuführbewegung wird durch ein Anschlagmittel 23 begrenzt, gegen das die Gegenstände 10 mit einer Stirnseite stoßen und welches sich seitlich entlang des Obertrums 18 in Förderrichtung weisend erstreckt.

Das Anschlagmittel 23 ist im vorliegenden Fall als Endlosförderer ausgebildet, wobei sich ein Fördertrum 15 des Endlosförderers entlang eines entsprechenden Randes des Obertrums 18 erstreckt und als seitlicher Anschlag für die Gegenstände 10 dient. Als Besonderheit können die Gegenstände 10 im Bereich des Fördertrums 15 mit einem Unterdruck beaufschlagt werden. Hierzu erstreckt sich auf der von den Gegenständen 10 abgewandten Seite des Fördertrums 15 ein Saugkasten 22 entlang des Fördertrums 15. Der Saugkasten 22 kann mit einer Unterdruckquelle verbunden und dazu eingerichtet sein über die entsprechende Länge des Fördertrums 15 einen Unterdruck auf der den Gegenständen 10 zugewandten Seite des Fördertrums 15 zu erzeugen. Das Fördertrum 15 kann hierzu entsprechend eingerichtet sein. Auf diese Weise wird verhindert, dass die Gegenstände 10 durch zu starkes Anprallen an einem seitlichen Anschlagmittel zurück in den Förderweg geschleudert werden.

Eine Besonderheit besteht in einem speziellen Förderer 24 zum Transport der Gegenstände 10 im Bereich der Zuführung derselben zum Gruppierförderer 12.

Der Förderer 24 ist um eine aufrechte Achse 25 mittels eines Antriebs 27 drehend antreibbar und weist eine Mantelfläche 26 auf, die zum Transport der Gegenstände 10 entsprechend deren Zuführung ausgerichtet ist.

Im vorliegenden Fall werden die Gegenstände 10 aufrecht stehend zugeführt und liegen tangential an der Mantelfläche 26 des Förderers 24 an, wodurch sie in Transportrichtung des Zuführförderers 19 weitertransportiert werden können. Weiterhin ist der Förderer 24 gleichzeitig zum seitlichen Transport der Gegenstände 10 in Förderrichtung des Gruppierförderers 12 eingerichtet. Zu diesem Zweck ist der Förderer 24 kegelförmig, insbesondere kegelstumpfförmig ausgebildet, mit der im wesentlichen aufrecht umlaufenden Mantelfläche 26. Zum seitlichen Transport der zugeführten Gegenstände 10 ist der Querschnitt des Förderers 24 ungleichmäßig ausgebildet, nämlich mit einem über den Umfang des Querschnitts sich veränderndem Radius. Auf diese Weise werden die Gegenstände 10 bei Anlage an der Mantelfläche 26 durch den zunehmenden Radius seitlich in Förderrichtung des Gruppierförderers 12 bewegt. Die Anordnung des Förderers 24 kann dabei so gewählt sein, dass eine sich bereits in einem Fach 13 auf dem Gruppierförderer 12 befindliche Produktgruppe 11 in Förderrichtung komprimiert wird.

Die Achse 25 des Förderers 24 ist im vorliegenden Fall in zwei Richtungen zur Senkrechten geneigt ausgebildet, sodass die Mantelfläche 26 im Kontaktbereich mit den aufrecht zu transportierenden Gegenständen 10 parallel zu diesen ausgerichtet ist unter tangentialer Ausrichtung der Gegenstände 10 zum Umfang des Förderers 24.

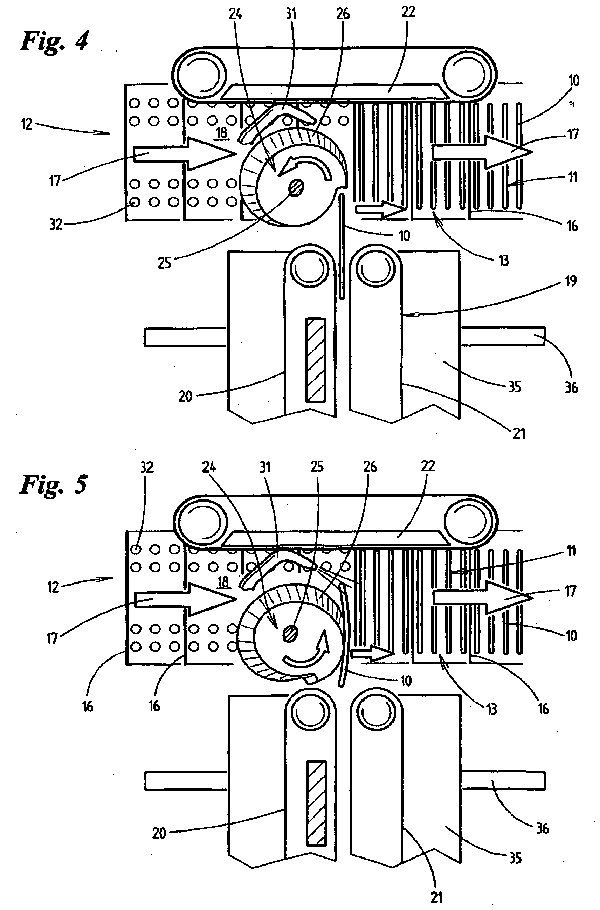

Wie bereits geschildert variiert der Radius des Förderers 24 über dem Umfang desselben. Der Radius kann sich kontinuierlich verändern, also zunächst kontinuierlich zunehmen und dann wieder auf einen anfänglichen Radius abnehmen. Alternativ kann der Radius wie im vorliegenden Fall kontinuierlich zunehmen um sich dann im Bereich eines Absatzes sprunghaft bzw. plötzlich zu verringern. Auf diese Weise kann ein Absatz in der Mantelfläche 26 gebildet sein. Die Aufgabe des Absatzes kann darin bestehen als Anschlag für die zugeführten Gegenstände 10 zu dienen. Alternativ ist es auch denkbar, dass der Absatz dazu dient zum seitlichen Abtransport der Gegenstände 10 in Förderrichtung des Gruppierförderers 12 beizutragen. Im ersteren Fall ist es denkbar, dass die Drehgeschwindigkeit des Förderers 24 geringer als die Zuführgeschwindigkeit der Gegenstände 10 ist, sodass die Gegenstände 10 an der durch den Absatz gebildeten Kante kontrolliert geführt werden (Fig. 4). Im letzteren Fall ist es denkbar, dass die Drehgeschwindigkeit des Förderers 24 größer als die Zuführgeschwindigkeit der Gegenstände 10 ist, sodass die Gegenstände 10 nicht gegen den Absatz stoßen, sondern durch diesen seitlich in Förderrichtung des Gruppierförderers 10 beschleunigt werden (Fig. 5).

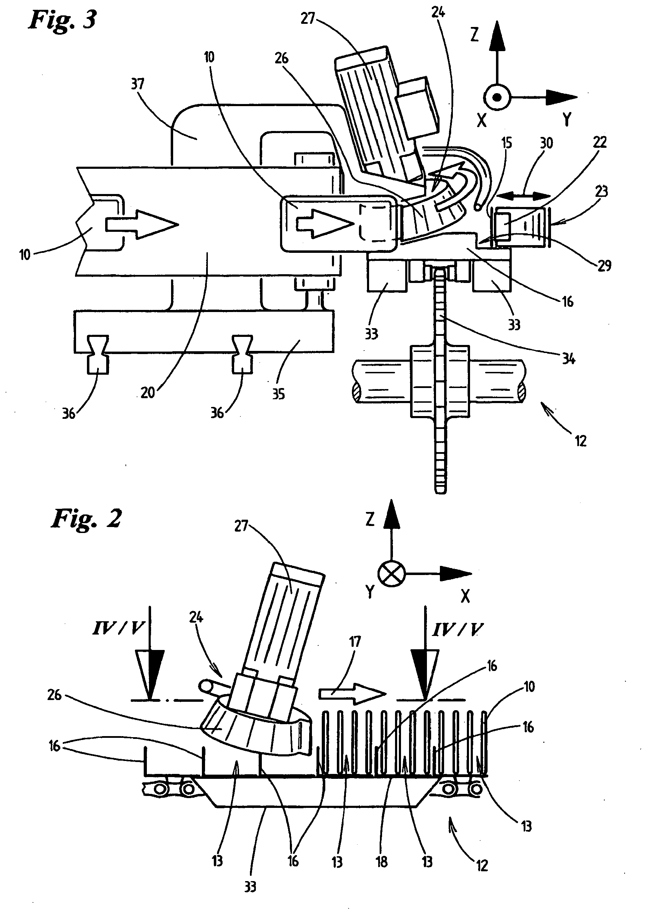

Wie aus Fig. 3 ersichtlich, ist der Förderer 24 oberhalb der Mitnehmer 16 angeordnet. Dabei sind die Mitnehmer 16 hinsichtlich Ihrer Ausbildung an die Ausrichtung des Förderers 24 besonders angepasst. Zum einen fällt die Oberseite der Mitnehmer 16 in Richtung auf den Zuführförderer 19 ab. Auf diese Weise wird die Neigung der Drehachse 25 berücksichtigt. Weiterhin ist die Höhe der Mitnehmer 16 im Bereich der Zuführung der Gegenstände 10 auf diese Weise so gering, dass eine unbeabsichtigte Kollision der Gegenstände 10 mit einem Mitnehmer 16 keine Verstopfung der Zufuhr in diesem Bereich bewirkt. Vielmehr bieten die Mitnehmer 16 allenfalls eine derart kleine Anschlagkante, dass die Gegenstände 10 seitlich an den Mitnehmern 16 vorbeigleiten.

Weiterhin zeigt Fig. 3, dass die Höhe der Mitnehmer 16 (deutlich) geringer als die Höhe der Gegenstände 10 ist, sodass diese oberhalb der Mitnehmer 16 durch den Förderer 24 transportiert werden können.

Im Bereich des Anschlagmittels 23 weisen die Mitnehmer 16 zudem eine Ausnehmung 29 auf, in deren Bereich das Fördertrum 15 bzw. der Saugkasten 22 verlaufen. Der Abstand des Anschlagmittels 23 im Bezug auf eine Längsmittelachse des Gruppierförderers 12 ist verstellbar ausgebildet entsprechend Doppelpfeil 30 (Fig. 2).

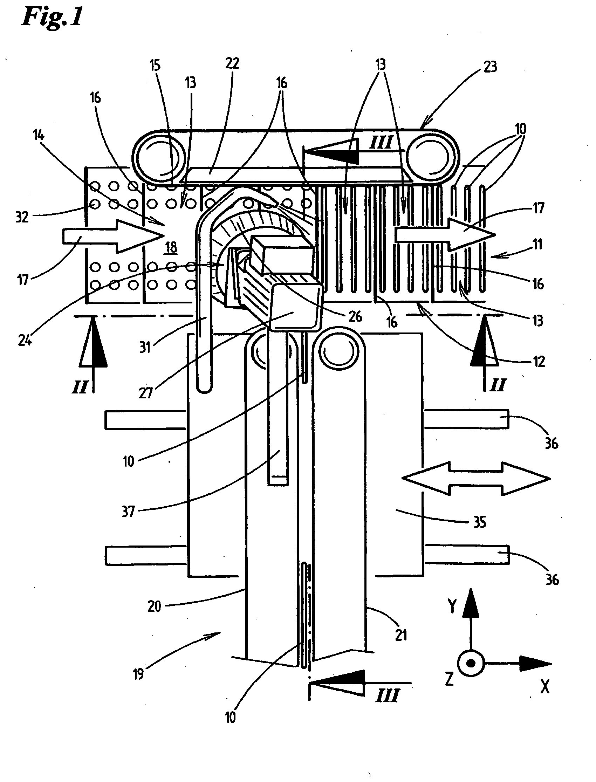

Die Gruppierung der Gegenstände 10 in mindestens einem Fach 13 des Gruppierförderers 12 kann als weitere Besonderheit durch Druckluft unterstützt werden. Hierzu ist im Arbeitsbereich des Förderers 24 eine Blaseinrichtung 31 vorgesehen. Die Blaseinrichtung 31 weist eine Düse auf, durch die Druckluft seitlich gegen die in den Fächern 13 befindlichen Gegenstände 10 geblasen werden kann (Fig. 1). Auf diese Weise werden die Gegenstände 10 gegen ein Umfallen gesichert, wenn beispielsweise die Produktgruppe 11 das Fach 13 nicht ausfüllt. Als Ursache denkbar ist beispielsweise eine planmäßig kleinere Produktgruppe 11 oder eine Störung in der Zuführung der Gegenstände 10. Weiterhin kann die Blaseinrichtung 31 auch den seitlichen Transport eines zugeführten Gegenstands 10 durch den Förderer 24 unterstützen (Fig. 5).

Zur Lagesicherung der Gegenstände 10 auf dem Obertrum 18 des Gruppierförderers 12 tragen zum einen die Mitnehmer 16 bei, die die Fächer 13 quer zur Transportrichtung des Gruppierförderers 12 begrenzen. Weiterhin trägt hierzu der im Bereich des Anschlagmittels 23 ausgeübte Unterdruck auf die Stirnseiten der Gegenstände 10 bei. Ein weiterer Beitrag erfolgt durch eine entsprechende Unterdruckeinrichtung im Bereich des Gruppierförderers 12. Hierzu weist dieser Durchbrüche 32 im Bereich des Obertrums 18 auf, über die durch unterhalb des Obertrums 18 angeordnete Saugkästen 33 die Gegenstände gegen das Obertrum 18 gesaugt werden.

Im vorliegenden Fall ist der Gruppierförderer 12 als Taschenkettenförderer ausgebildet, der auch die Mitnehmer 16 bildet. Unterhalb des Obertrums 18 ist der Taschenkettenantrieb 34 geführt, wobei beiderseits des Antriebs sich die Saugkästen 33 in Längsrichtung des Gruppierförderers 13 erstrecken.

Konstruktiv sind Zuführförderer 19, Förderer 24 und Blaseinrichtung 31 zu einer gemeinsam bewegbaren Einheit verbunden. Die Einheit ist auf einem Schlitten 35 gelagert, der seitlich auf Schienen 36 verfahrbar gelagert ist, nämlich parallel zur Förderrichtung des Gruppierförderers 12. Der Förderer 24 mit Antrieb 27 ist über einen Bügel 37 mit dem Schlitten 35 verbunden. Bei fehlender Zuführung der Gegenstände 10 zum Gruppierförderer 12 kann die Einheit seitlich verfahren werden, um die Lücken im Förderstrom auszugleichen.

Nach Bildung einer Produktgruppe 11 in einem Fach 13 kann die Produktgruppe 11 durch eine nicht gezeigte Abschubeinrichtung aus dem Fach 13 des Gruppierförderers 12 abgefördert werden. Hierzu kann die Abschubeinrichtung 30 einen Schieber aufweisen, der winklig, vorzugsweise quer, zur Förderrichtung des Gruppierförderers 12 bewegbar ist, um zwischen zwei Mitnehmern 16 hindurch eine Produktgruppe 11 zu erfassen und quer vom Gruppierförderer 12 abzuschieben. Da der Gruppierförderer 12 kontinuierlich angetrieben wird, kann die Abschubbewegung des Schiebers mit einer in Förderrichtung des Gruppierförderers 12 weisenden Bewegung überlagert werden.

Mit Hilfe einer nicht gezeigten Steuerung sind die Antriebe des Förderers 24 (Antrieb 27), und/oder des Gruppierförderers 12 (Antrieb 34) und/oder des Schlittens (nicht gezeigt) und/oder der Abschubeinrichtung aufeinander abgestimmt.

### Bezugszeichenliste

- 10: Gegenstand
- 11: Produktgruppe
- 12: Gruppierförderer
- 13: Fach
- 14: Fördertrum
- 15: Fördertrum (Anschlagmittel)
- 16: Mitnehmer
- 17: Pfeil
- 18: Obertrum
- 19: Zuführförderer
- 20: Endlosförderer
- 21: Endlosförderer
- 22: Saugkasten
- 23: Anschlagmittel
- 24: Förderer
- 25: Achse
- 26: Mantelfläche
- 27: Antrieb (Förderer)
- 29: Ausnehmung
- 30: Doppelpfeil
- 31: Blaseinrichtung
- 32: Durchbruch
- 33: Saugkasten
- 34: Taschenkettenantrieb
- 35: Schlitten
- 36: Schiene
- 37: Bügel

## Patentansprüche

1. Vorrichtung zum Gruppieren von flachen Gegenständen (10), insbesondere von Hygieneprodukten wie Windeln, wobei die zu gruppierenden Gegenstände (10) einzeln und nacheinander auf einem Zuführförderer (19) transportierbar und einem Gruppierförderer (12) zuführbar sind, **dadurch gekennzeichnet, dass** der Gruppierförderer (12) Fächer (13) für jeweils eine Gruppe (11) von Gegenständen (10) aufweist, in denen die Gegenstände (10) vorzugsweise aufrecht stehend, insbesondere in Dichtlage angeordnet sind, wobei die Gegenstände (10) winklig, insbesondere im Wesentlichen quer, zur Förderrichtung des Gruppierförderer (12) durch den Zuführförderer (19) zuführbar sind und mit einem im Wesentlichen kegelförmigen Förderer (24) in Transportrichtung des Gruppierförderers (12) förderbar sind unter Bildung einer Gruppe (11) von Gegenständen (10) in einem Fach (13), wobei der im Wesentlichen kegelförmige Förderer (24) um eine aufrechte Drehachse (25) drehend antreibbar ist und die Gegenstände (10) während des Transports durch den im Wesentlichen kegelförmigen Förderer (24) im Bereich einer Mantelfläche (26) des im Wesentlichen kegelförmigen Förderers (24) anliegen, wobei die Drehachse (25) in zwei Richtungen zur Senkrechten geneigt ist, derart, dass der Bereich der Mantelfläche (26), an dem die Gegenstände (10) zum Transport anliegen, im Wesentlichen quer zur Transportbahn der Gegenstände (10), insbesondere senkrecht, gerichtet ist und die Transportbahn der Gegenstände (10) im Wesentlichen tangential zur Mantelfläche (26) des im Wesentlichen kegelförmigen Förderers (24) verläuft, und dass die Drehachse (25) exzentrisch angeordnet ist, derart, dass die Gegenstände (10) bei Drehung des Förderers (24) winklig, insbesondere quer, zur Förderrichtung des Zuführförderers (19) bzw. im Wesentlichen in Transportrichtung des Gruppierförderers (12) förderbar sind.

2. Vorrichtung nach Anspruch 1, **dadurch gekennzeichnet, dass** im Bereich der Mantelfläche (26) des Förderers (24) ein Absatz für die Gegenstände (10) ausgebildet ist zur Anlage einer in Förderrichtung vorne liegenden Kante der Gegenstände (10) bei der Zuführung zum Gruppierförderer (12).

3. Vorrichtung nach Anspruch 1 oder einem der weiteren vorhergehenden Ansprüche, **dadurch gekennzeichnet, dass** die Umfangsgeschwindigkeit des drehend angetriebenen Förderers (24) größer als die Zuführgeschwindigkeit der Gegenstände (10) ist.

4. Vorrichtung nach Anspruch 1 oder einem der weiteren vorhergehenden Ansprüche, **dadurch gekennzeichnet, dass** im Arbeitsbereich des Förderers (24) eine Einrichtung (31) zur Abgabe eines seitlich in Förderrichtung des Gruppierförderers (12) auf die Gegenstände (10) wirkenden Luftstroms positioniert ist, insbesondere eine Blaseinrichtung (31).

5. Vorrichtung nach Anspruch 1 oder einem der weiteren vorhergehenden Ansprüche, **dadurch gekennzeichnet, dass** die Gegenstände (10) auf einem Fördertrum (18) des Gruppierförderers (12) ruhend transportierbar sind, wobei das Fördertrum (18) zur Beaufschlagung der Gegenstände (10) mit einem Unterdruck eingerichtet ist.

6. Vorrichtung nach Anspruch 1 oder einem der weiteren vorhergehenden Ansprüche, **dadurch gekennzeichnet, dass** der Gruppierförderer (12) wenigsten im Bereich der Zuführung der Gegenstände (10) ein quer zur Transportrichtung der Gegenstände (10) auf dem Zuführförderer (19) gerichtetes Anschlagmittel (23) aufweist, wobei das Anschlagmittel (23) zur Beaufschlagung der Gegenstände (10) mit einem Unterdruck eingerichtet ist.

7. Vorrichtung nach Anspruch 1 oder einem der weiteren vorhergehenden Ansprüche, **dadurch gekennzeichnet, dass** ein ausgabeseitiges Ende des Zuführförderers (19), der Förderer (24) und die Blaseinrichtung (31) zu einer gemeinsam bewegbaren Einheit verbunden sind, die parallel zum Gruppierförderer (12) seitlich bewegbar ist.

8. Vorrichtung nach Anspruch 1 oder einem der weiteren vorhergehenden Ansprüche, **dadurch gekennzeichnet, dass** die auf dem Gruppierförderer (12) gebildeten Fächer (13) durch Mitnehmer (16) des Fördertrums (18) begrenzt sind, wobei die Gegenstände (10) über die Oberseite der Mitnehmer (16) hinausragen und der Förderer (24) zum Transport der Gegenstände (10) oberhalb der Mitnehmer (16) angeordnet ist.

9. Vorrichtung nach Anspruch 1 oder einem der weiteren vorhergehenden Ansprüche, **dadurch gekennzeichnet, dass** eine Oberseite der Mitnehmer (16) entgegen der Zuführrichtung der Gegenstände (10) auf dem Gruppierförderer (12) abfallend geneigt ausgebildet ist.

## Claims

1. A device for grouping flat articles (10), in particular hygiene products such as diapers, wherein the articles (10) to be grouped are conveyable individually and one after another on a feed conveyor (19) and are suppliable to a grouping conveyor (12), **characterized in that** the grouping conveyor (12) has compartments (13) for in each case one group (11) of articles (10), in which compartments the articles (10) are arranged preferably standing upright, in particular tightly packed together, wherein the articles (10) are suppliable by means of the feed conveyor (19) at an angle, in particular substantially transversely, with respect to the conveying direction of the grouping conveyor (12) and are conveyable in the conveying direction of the grouping conveyor (12) by way of a substantially cone-shaped conveyor (24) thus forming a group (11) of articles (10) in one compartment (13), wherein the substantially cone-shaped conveyor (24) is rotatingly driveable about driveable about an upright axis of rotation (25) and, when being conveyed by the substantially cone-shaped conveyor (24), the articles (10) sit in the region of an outside surface (26) of the substantially cone-shaped conveyor (24), wherein the axis of rotation (25) is inclined with respect to the vertical in two directions, that is to say in such a manner that the region of the outside surface (26), against which the articles (10) abut for conveying, is directed substantially transversely, in particular vertically, with respect to the conveying path of the articles (10) and the conveying path of the articles (10) extends substantially tangentially with respect to the outside surface (26) of the substantially cone-shaped conveyor (24) and **in that** the axis of rotation (25) is arranged in an eccentric manner such that, when the conveyor (24) rotates, the articles (10) are conveyable at an angle, in particular transversely, with respect to the conveying direction of the feed conveyor (19) or substantially in the conveying direction of the grouping conveyor (12).

2. The device as claimed in claim 1, **characterized in that** a shoulder for the articles (10) is realized in the region of the outside surface (26) of the conveyor (24) for the abutment of an edge of the articles (10) lying in front in the conveying direction when they are being supplied to the grouping conveyor (12).

3. The device as claimed in claim 1 or in one of the other preceding claims, **characterized in that** the circumferential speed of the rotatingly driven conveyor (24) is faster than the speed the articles (10) are supplied.

4. The device as claimed in claim 1 or in one of the other preceding claims, **characterized in that** a device (31) for delivering an air flow which acts on the articles (10) laterally in the conveying direction of the grouping conveyor (12) is positioned in the operating region of the conveyor (24), in particular a blowing device (31).

5. The device as claimed in claim 1 or in one of the other preceding claims, **characterized in that** the articles (10) are conveyable in a stationary manner on a conveyor run (18) of the grouping conveyor (12), wherein the conveyor run (18) is set up to act upon the articles (10) with negative pressure.

6. The device as claimed in claim 1 or in one of the other preceding claims, **characterized in that,** at least in the region of the of the feeding of the articles (10), the grouping conveyor (12) has a stop means (23) which is directed transversely with respect to the conveying direction of the articles (10) on the feed conveyor (19), wherein the stop means (23) is set up to act upon the articles (10) with negative pressure.

7. The device as claimed in claim 1 or in one of the other preceding claims, **characterized in that** an output-side end of the feed conveyor (19), the conveyor (24) and the blowing device (31) are connected to form one unit which is movable as one and which is movable laterally parallel to the grouping conveyor (12).

8. The device as claimed in claim 1 or in one of the other preceding claims, **characterized in that** the compartments (13) formed on the grouping conveyor (12) are defined by entrainment means (16) of the conveyor run (18), wherein the articles (10) project beyond the top surface of the entrainment means (16) and the conveyor (24) is arranged above the entrainment means (16) for conveying the articles (10).

9. The device as claimed in claim 1 or in one of the other preceding claims, **characterized in that** a top surface of the entrainment means (16) is realized so as to be inclined in a descending manner on the grouping conveyor (12) in opposition to the direction in which the articles (10) are supplied.

## Revendications

1. Dispositif pour grouper des objets plats (10), en particulier des produits hygiéniques tels que des couches, dans lequel les objets à grouper (10) peuvent être transportés un à un et l'un derrière l'autre sur un transporteur d'alimentation (19) et fournis à un transporteur de groupage (12), **caractérisé en ce que** le transporteur de groupage (12) présente des cases (13) destinées chacune à un groupe (11) d'objets (10), dans lesquelles les objets (10) sont disposés de préférence en position dressée, en particulier en position serrée, dans lequel les objets (10) peuvent être fournis par le transporteur d'alimentation (19) sous un angle, en particulier essentiellement à angle droit, par rapport à la direction de transport du transporteur de groupage (12) et ils peuvent être transportés avec un transporteur essentiellement conique (24) dans la direction de transport du transporteur de groupage (12) en formant un groupe (11) d'objets (10) dans une case (13), dans lequel le transporteur (24) essentiellement conique peut être entraîné en rotation autour d'un axe de rotation dressé (25) et les objets (10) reposent dans la région d'une surface latérale (26) du transporteur (24) essentiellement conique pendant le transport par le transporteur (24) essentiellement conique, dans lequel l'axe de rotation (25) est incliné dans deux directions par rapport à la verticale, de telle manière que la région de la surface latérale (26), sur laquelle les objets (10) sont déposés pour le transport, est orientée essentiellement transversalement à la voie de transport des objets (10), en particulier perpendiculairement, et la voie de transport des objets (10) s'étend pour l'essentiel tangentiellement à la surface latérale (26) du transporteur (24) essentiellement conique, et **en ce que** l'axe de rotation (25) est disposé en position excentrique, de telle manière que les objets (10) puissent, par rotation du transporteur (24), être transportés sous un angle, en particulier transversalement, par rapport à la direction de transport du transporteur d'alimentation (19) ou essentiellement dans la direction de transport du transporteur de groupage (12).

2. Dispositif selon la revendication 1, **caractérisé en ce qu'**un épaulement pour les objets (10) est formé dans la région de la surface latérale (26) du transporteur (24) pour l'appui d'une arête des objets (10) située en avant dans la direction de transport lors de la fourniture au transporteur de groupage (12).

3. Dispositif selon la revendication 1 ou l'une quelconque des autres revendications précédentes, **caractérisé en ce que** la vitesse périphérique du transporteur (24) entraîné en rotation est plus grande que la vitesse de fourniture des objets (10).

4. Dispositif selon la revendication 1 ou l'une quelconque des autres revendications précédentes, **caractérisé en ce qu'**un dispositif (31) pour la délivrance d'un courant d'air agissant sur les objets (10) latéralement dans la direction de transport du transporteur de groupage (12) est positionné dans la région de travail du transporteur (24), en particulier un dispositif de soufflage (31).

5. Dispositif selon la revendication 1 ou l'une quelconque des autres revendications précédentes, **caractérisé en ce que** les objets (10) peuvent être transportés au repos sur un brin de transport (18) du transporteur de groupage (12), dans lequel le brin de transport (18) est conçu de façon à soumettre les objets (10) à une dépression.

6. Dispositif selon la revendication 1 ou l'une quelconque des autres revendications précédentes, **caractérisé en ce que** le transporteur de groupage (12) présente, au moins dans la région de la fourniture des objets (10), un moyen de butée (23) orienté transversalement à la direction de transport des objets (10) sur le transporteur d'alimentation (19), dans lequel le moyen de butée (23) est conçu de façon à soumettre les objets (10) à une dépression.

7. Dispositif selon la revendication 1 ou l'une quelconque des autres revendications précédentes, **caractérisé en ce qu'**une extrémité côté délivrance du transporteur d'alimentation (19), le transporteur (24) et le dispositif de soufflage (31) sont réunis en une unité mobile commune, qui est déplaçable latéralement parallèlement au transporteur de groupage (12).

8. Dispositif selon la revendication 1 ou l'une quelconque des autres revendications précédentes, **caractérisé en ce que** les cases (13) formées sur le transporteur de groupage (12) sont limitées par des éléments d'entraînement (16) du brin de transport (18), dans lequel les objets (10) sont proéminents au-dessus du côté supérieur des éléments d'entraînement (16) et le transporteur (24) destiné au transport des objets (10) est disposé au-dessus des éléments d'entraînement (16) .

9. Dispositif selon la revendication 1 ou l'une quelconque des autres revendications précédentes, **caractérisé en ce qu'**un côté supérieur des éléments d'entraînement (16) présente une pente descendante à l'encontre de la direction de transport des objets (10) sur le transporteur de groupage (12).
